# EUROPEAN PATENT APPLICATION

(11) **EP 2 359 810 A2**
(43) Date of publication of application: **24.08.2011**
(21) Application number: 11003617.5
(22) Date of filing: 17.09.2007
(51) Int. Cl.: A61K 9/16, A61K 9/20, A61K 31/60

(54) **Pharmaceutical formulations comprising clopidogrel**

(30) Priority: 16.09.2006 EP 06019429
(62) Divisional of application: 07818184.9
(71) Applicant: Acino Pharma AG, 4058 Basel (CH)
(72) Inventor: Rey, Hélène, 68128 Rosenau (FR); Fischer, Marc, 4106 Therwil (CH); Scheer, Mathias, 79664 Wehr (DE)
(74) Representative: Breuer, Markus

(57) **Abstract**

Melt granulate in the form of a solid state dispersion of the drug in a pharmaceutically acceptable carrier, wherein said melt granulate comprises a pharmaceutically active compound, preferably clopidogrel, at least one carrier material, and optionally pharmaceutically acceptable excipients and method of making said melt granulate.

## Description

The present invention relates to certain pharmaceutical formulations comprising clopidogrel or a pharmaceutically acceptable salt thereof, processes for their manufacture and their use in a method of treating warm-blooded animals, including preferably mammals and especially humans.

### BACKGROUND OF THE INVENTION

Clopidogrel is methyl (+)-(S)-alpha-(2-chlorophenyl)-4,5,6,7-tetrahydrothieno[3,2-c]pyridine-5-acetate (= d-enantiomer) of the following formula:

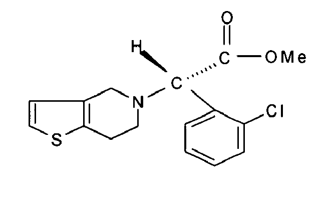

It is marketed in the form of its bisulfate by Sanofi under the trade name PLAVIX® (INN: clopidogrel bisulfate), inter alia as an antiplatelet drug.

The racemic form of clopidogrel of the formula 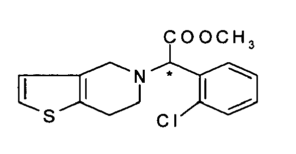 in which the C* is an asymmetric carbon atom in both (R)- and (S)-configuration, and its pharmaceutically acceptable salts are described in US 4,529,596 and EP 099 802.

The dextro-rotatory enantiomer (= d-enantiomer) of methyl alpha-5-(4,5,6,7-tetrahydro(3,2-c)thieno-pyridyl)-(2-chlorophenyl)-acetate and certain of its pharmaceutically acceptable salts, like especially its bisulfate, are described in US 4,847,265 and EP 0 281 459.

According to US 4,847,265 many salts of clopidogrel have the drawback of being hygroscopic and difficult to handle on an industrial scale, and their purification proved to be difficult. EP 1 310 245 mentions that magnesium stearate should not be used as a lubricant together with clopidogrel because generally there is an interaction between clopidrogrel bisulphate and magnesium stearate causing degradation and instability of clopidogrel tablets which contain magnesium stearate.

### DESCRIPTION OF THE INVENTION

It has now surprisingly been found how the above-mentioned drawback can be overcome. According to the present invention clopidogrel and its pharmaceutically acceptable acid addition salts can be stabilized in pharmaceutical formulations comprising melt granulates.

As shown in the Detailed Description of Experiments hereinafter, the stabilizing effect of the melt granulate formulations is so pronounced that it allows using also those pharmaceutically acceptable acid addition salts of clopidogrel which have been described in US 4,847,265 as being unsuitable, e.g. the salt with benzenesulfonic acid, i.e. the besylate. Even more surprisingly, the stabilizing effect of the melt granulate formulations is especially pronounced for clopidogrel salt batches of bad quality. This is especially valuable for salts the purification of which proves to be difficult. For example, as evident from Table 2 in Example 10 herein clopidogrel hydrochloride contains after storage for 30 days at 40°C and 75% relative humidity nearly twice as much impurities than the clopidogrel hydrochloride stabilized in the melt granulates of the present invention. Hence, the formation of the melt granulates of the present invention surprisingly markedly reduces degradation of clopidogrel and its salts on storage.

The acid addition salts of clopidogrel which can be stabilized according to the present invention are those with pharmaceutically acceptable mineral or organic acids classically used in pharmacy. Appropriate acids are, for example, inorganic acids, such as hydrohalic acid, e. g. hydrochloric, hydrobromic or the like, or sulfuric acid, nitric acid, or phosphoric acid; or suitable organic acids, for example suitable aliphatic acids, like aliphatic mono or dicarboxylic acids, hydroxyalkanoic or hydroxyalkanedioic acids, e.g. acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxy-butanedioic, 2,3-dihydroxybutanedioic, or 2-hydroxy-1,2,3-pro-panetricarboxylic acid; phenylsubstituted alkanoic acids; or suitable aromatic acids, like 2-hydroxybenzoic, or 4-amino-2-hydroxybenzoic acid; or suitable sulfonic acids, like alkanesulfonic acids, e.g. methanesulfonic, or ethanesulfonic acid, or aromatic sulfonic acids, e.g. benzenesulfonic, or 4-methylbenzenesulfonic acid; or cyclohexanesulfamic acid. Preferred acids are e.g. hydrobromic acid, sulphuric acid, phosphoric acid, acetic, benzoic, fumaric, maleic, citric, tartaric, gentisic, dobesilic, methanesulfonic, ethanesulfonic, lauryl-sulfonic, and para-toluenesulfonic acids, especially hydrochloric acid, and most especially benzenesulfonic acid.

The term "pharmaceutically acceptable salt" as used herein refers to salts which are known to be non-toxic and are commonly used in the pharmaceutical literature. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, beta hydroxybutyrate, chloride, cinnamate, citrate, formate, fumarate, glycolate, heptanoate, lactate, maleate, hydroxymaleate, malonate, nitrate, oxalate, phthalate, phosphate, monohydrogenphosphate, dihydrogenphosphate, metaphosphate, pyrophosphate, propionate, phenylpropionate, salicylate, succinate, sulfate, bisulfate (hydrogensulfate), pyrosulfate, sulfite, bisulfite, sulfonate, p-bromophenylsulfonate, chlorobenzenesulfonate, ethanesulfonate, 2-hydroxyethanesulfonate, methanesulfonate (mesylate), naphthalene-1- sulfonate, naphthalene-2-sulfonate, p-toluenesulfonate (tosylate), xylenesulfonate, tartarate, and the like. Preferred salts are the hydrochloride (chloride) and the benzenesulfonate (besylate).

These salts are prepared in a standard manner, for example by the action of the corresponding acid on clopidogrel base in solution in a solvent from which they precipitate spontaneously or after addition of a non-solvent of the salt.

The term "melt granulate" as used herein defines a solid state dispersion of the active ingredient, i.e. clopidogrel or its pharmaceutically acceptable acid addition salts, i.e. the "drug", in an inert or pharmaceutically acceptable carrier or matrix prepared by a melting (fusion) method. The dispersion of the active ingredient in a solid carrier or diluent by traditional mechanical mixing is not included within the definition of this term.

The term "pharmaceutically acceptable excipients" as used herein defines the excipients which may be added to the melt or later to the solid state dispersion of the drug in the pharmaceutically acceptable carrier, i.e. to the melt granulate, e.g. for tabletting.

As used herein, the term "pharmaceutically acceptable carrier" refers preferably to suitable polyethylene glycols (PEGs) as defined herein below. Further carriers which can be used in accordance with the present invention include e.g. Compritol 888 ATO. Compritol [trade mark] is designated in The National Formulary (NF) as glyceryl behenate, and according to the International Nomenclature of Cosmetic Ingredients [INCI] as Tribehenin. It is obtainable for example from the company Gatte-fossé. Compritol is a mixture of esters of glycerol with C₁₆-C₂₂ fatty acids, mainly with C₂₂ fatty acid, i.e. behenic acid.

Polyethylene glycols, also called macrogols, are manufactured by polymerization of ethylene oxide with either water, monoethylene glycol or diethylene glycol under alkaline catalysis. After the desired molecular weight is reached (according to viscosity measurements) the reaction is terminated by neutralizing the catalyst with an acid. The result are molecules of the structure HO-[CH₂-CH₂-O]ₙ-H wherein n is the number of ethylene oxide units.

As an abbreviation for polyethylene glycols the term "PEG" is used in combination with a numerical value which, within the pharmaceutical industry, indicates the mean molecular weight. Unfortunately, the various Parmacopeias use different nomenclature for some PEG molecular weights. The molecular weights cited herein are in accordance with the European (2002) and US Pharmacopeias. The hardness of PEGs increases with increasing molecular weight, however the melting range goes up to a maximum value of about 60°C only. The most important property of all PEGs is their solubility in water. The lower PEGs up to a molecular weight below about 2000 are hygroscopic. PEGs can be obtained for example from the company Clariant, Switzerland. Suitable PEGs for the manufacture of melt granulates according to the present invention have a molecular weight from about 1500 to 35000. Preferred are PEGs having a molecular weight from about 2000 to 20000, especially from about 4000 to 20000, more especially from about 4000 to 10000, e.g. 6000.

In this sense, the present invention provides a melt granulate, i.e. a pharmaceutical composition in the form of a solid state dispersion of the drug in a pharmaceutically acceptable carrier, said melt granulate comprising a pharmaceutically active compound, i.e. clopidogrel, at least one carrier material, and optionally pharmaceutically acceptable excipients.

Usually, the general method for preparation of a solid dispersion proceeds by a fusion process wherein a pharmaceutically acceptable carrier is mixed with the drug to form an intimate mixture. The mixture is heated at or near the temperature of the melting point of the pharmaceutically acceptable carrier, thus forming a melt. However, the mixture is heated sufficiently below the melting point of the pharmaceutically active compound, i.e. clopidogrel, in order to avoid any decomposition of the compound. The molten mixture is then cooled rapidly to provide a congealed mass which may subsequently be milled to produce a powder.

Alternatively, the pharmaceutically acceptable carrier can be heated to molten condition upon which the drug can be added to the molten carrier or vice versa under stirring or mixing, e.g. in a high shear mixer, like a Diosna mixer, thus forming a molten homogeneous melt. Preferably, the molten carrier is added to the drug under stirring or mixing.

Lastly, the process proceeds by cooling the molten homogeneous melt to form a solid state dispersion. Cooling can be effected by conventional methods or, according to the preferred method of the present invention, by shock cooling, for example by adding, in a high shear mixer, dry ice, i.e. solid carbon dioxide having a temperature of about -78°C, preferably in the form of a kind of snow ("dry snow"), or liquefied air, preferably solid carbon dioxide, to the dispersion which thereupon breaks apart into many small particles which are sieved, e.g. through 1-2 mm. If cooling is carried out slowly, the average particle size obtained generally is too large so that subquent milling of the product is required. It was surprising to find that the addition of solid carbon dioxide or liquefied air to the melt yielded the required average particle size without destabilizing the active product.

The present invention refers to a method of making a pharmaceutical composition in the form of a solid state dispersion of a pharmaceutically active compound (drug) in a pharmaceutically acceptable carrier in the form of a melt granulate, said melt granulate comprising a pharmaceutically active compound, preferably clopidogrel, at least one carrier material, and optionally pharmaceutically acceptable excipients, characterized in that the pharmaceutically acceptable carrier is mixed with a pharmaceutically active compound, and optionally also with the pharmaceutically acceptable excipients, to form an intimate mixture, the obtained mixture is then heated at or near the temperature of the melting point of the pharmaceutically acceptable carrier but below the melting point of the pharmaceutically active compound, thus forming a melt, the obtained melt is then cooled rapidly to provide a congealed mass which optionally is milled to produce a powder.

The present invention also refers to a method of making a pharmaceutical composition in the form of a solid state dispersion of a pharmaceutically active compound in a pharmaceutically acceptable carrier in the form of a melt granulate, said melt granulate comprising a pharmaceutically active compound, preferably clopidogrel, at least one carrier material, and optionally pharmaceutically acceptable excipients, characterized in that the pharmaceutically acceptable carrier, optionally containing pharmaceutically acceptable excipients, is heated at or near the temperature of the melting point of the pharmaceutically acceptable carrier but below the melting point of the pharmaceutically active compound; the molten pharmaceutically acceptable carrier is then mixed with the pharmaceutically active compound until a homogeneous melt is formed; the obtained melt is then cooled rapidly to provide a congealed mass which optionally is milled to produce a powder. Preferably, the molten carrier is added to the drug under stirring or mixing.

The obtained melt is cooled rapidly. This cooling process proceeds by cooling the molten homogeneous melt to form a solid state dispersion. Said cooling can be effected by conventional methods. Preferred, however, is the cooling by a shock cooling, preferably by adding, preferably in a high shear mixer, dry ice, i.e. solid carbon dioxide, having a temperature of about -78°C, preferably in the form of a kind of snow ("dry snow"), or liquefied air, preferably solid carbon dioxide, to the dispersion which thereupon cools rapidly and breaks apart into many small particles which can be sieved, e.g. through 1-2 mm.

The ratio of the drug to the pharmaceutically acceptable carrier can be varied over a wide range and depends on the concentration of the drug required in the pharmaceutical dosage form ultimately administered. However, the preferred range of the drug in the solid dispersion is about 10% to 90% of the total solid dispersion weight, more preferable is about 20% to 90%, even more preferable is about 40% to 90%, especially about 50% to 90%, e.g. 50, 60, 70 or 80%, of the total dispersion weight.

Examples of pharmaceutically acceptable excipients include diluents resp. fillers (e.g. Pharmatose® DCL 11), binders, disintegrants (e.g. Explocel®), coloring agents, flavoring agents, lubricants (e.g. Cutina®HR, a hydrated castor oil, or e.g. magnesium stearate) and/or preservatives. The excipients may be added to the melt or later to the solid state dispersion of the drug, e.g. for tabletting resp. compressing.

The pharmaceutical composition may be formulated by conventional methods of admixture such as blending, filling, granulation and compressing.

Besides acting as carriers for the active ingredient, PEGs also act as lubricants and binders during the tablet processing and solid PEGs are also frequently used in tablet coatings. These other uses of PEGs are distinguishable and have to be clearly distinguished from the use in the formation of melt granulates.

The medicine of the invention can be made available for oral administration for example in the form of tablets, which may be coated, like e.g. sugar-coated, or capsules.

The melt granulates and pharmaceutical compositions of the present invention may be used to treat warm-blooded animals, including mammals and humans, for the same indications which can be treated by PLAVIX, e.g. on account of its interesting inhibitory properties towards platelet aggregation and its interference in the mechanism of formation of arterial and venous thromboses. The medicine of the invention can be usefully administered in the treatment and prevention of platelet disorders, including for example also those due to extracoporeal blood circuits or the consequence of complications in atheroma. Mainly, the melt granulates of the present invention may be used for the manufacture of pharmaceutical compositions for reduction of atherothrombotic events in case of recent myocardial infarction, recent stroke, established peripheral arterial disease, and acute coronary syndrome.

The composition is usually presented as a unit dose composition containing from 30 to 100 mg, more usually from 60 to 90 mg, preferably 75 mg of clopidogrel. Such composition is normally administered once daily to a warm-blooded animal, including a human, of about 70 kg body weight, or as recommended for PLAVIX. As usual, the exact dose to be administered depends inter alia on the age, health and weight of the recipient; the nature and extent of the symptoms; the kind of concurrent treatment; the frequency of treatment; and the effect desired. The treatment of patients with acute coronary syndrome should be initiated with a single 300 mg loading dose of clopidogrel and then continued at 75 mg once daily. In addition, Aspirin and/or heparin may be administered. The following examples illustrate the invention.

### Abbreviations:

rpm: rounds per minute
s: second(s)

### Example 1 (melt granulate consisting of 10 % by weight of PEG 6000 and 90% of clopidogrel besylate)

Melt 22.4 g of PEG 6000 using a hot plate, until it has reached 80°C. Place 201.3 g of clopidogrel besylate in a high-shear mixer. While stirring (400 rpm), pour the melted PEG into the bowl containing clopidogrel. Add 50 g of dry ice (in the form of dry snow) and increase the speed to 3000 rpm for 30 seconds. The resulting granulate has a temperature of about 15°C, and is sieved through 2.0 and 1.0 mm.

Clopidogrel besylate used as a starting material is prepared as follows:
Step 1: Manufacture of clopidogrel besylate 7 g (0.0228 mole) of dextro-rotatory methyl alpha-5(4,5,6,7-tetrahydro(3,2-c)thieno pyridyl) (2-chlorophenyl)acetate (i.e. clopidogrel in the form of its free base) is dissolved in 100 ml of diethyl ether. To the obtained solution 30 ml of diethyl ether containing 4.78 g (0.03 mole) of anhydrous benzene sulfonic acid are added at room temperature (i.e. at 28°C - 31°C). After complete addition the reaction mass is maintained at room temperature (28°C - 31°C) for 14 hours. The white solid is collected by filtration and dried at 50-60 °C in a vacuum oven whereupon 2.2 g of clopidogrel besylate are obtained in crystalline form (99.82% pure according to HPLC); DSC: onset at 127.45°C, peak at 135.69°C.

### Example 2 (melt granulate consisting of 30 % PEG 6000 and 70% clopidogrel besylate)

Analogously as described in Example 1 the above melt granulate is obtained from 86.4 g of clopidogrel besylate, 201.3 g of PEG 6000, and 80 g of dry snow.

### Example 3 (melt granulate consisting of 50 % PEG 6000 and 50% clopidogrel besylate)

Analogously as described in Example 1 the above melt granulate is obtained from 201.3 g of clopidogrel besylate and 201.3 g of PEG 6000, adding between 130 and 150 g of dry snow and increasing the speed of the mixer to 3000 rpm for 1 minute.

### Example 4 (melt granulate consisting of 50 % PEG 4000 and 50% clopidogrel besylate)

Melt 201.3 g of PEG 4000 using a hot plate, until it has reached 76 °C. Place 201.3 g of clopidogrel besylate in a high-shear mixer. While stirring (600 rpm), pour the melted PEG in the bowl containing the clopidogrel. Add 150 g of dry ice and increase the speed to 1800 rpm for 1 mn. The resulting granulate has a temperature of 17.6°C, and is sieved through 2.00 and 1.0 mm

### Example 5 (melt granulate consisting of 50 % PEG 20000 and 50% clopidogrel besylate)

201.3 g of PEG 20000 are melted using a hot plate, until it has reached 100°C. 201.3 g of clopidogrel besylate are placed into a high-shear mixer. While stirring (400 rpm) the melted PEG is poured into the bowl containing the clopidogrel. 50 g of dry ice are added and the speed of the mixer is increased to 3000 rpm for 1 minute. The resulting granulate has a temperature of 15°C, and is sieved through 2.0 and 1.0 mm.

### Example 6 (melt granulate consisting of 50 % by weight of PEG 35000 and 50% clopidogrel besylate)

Melt 201.3 g of PEG 35000 using a hot plate, until it has reached 100°C. Place 201.3 g of clopidogrel besylate in a high-shear mixer. While stirring (400 rpm), pour the melted PEG into the bowl containing the clopidogrel. Add 100 g of dry ice and increase the speed to 3000 rpm for 2 minutes. The resulting granulate has a temperature of 30°C, and is sieved through 2.0 and 1.0 mm.

### Example 7 (melt granulate consisting of 50 % by weight of PEG 6000 and 50% clopidogrel hydrochloride)

Melt 10.0 g of PEG 6000 using a 78°C water bath until it has reached 74°C. Place 10.0 g of clopidogrel hydrochloride into a mortar. While manually stirring, pour the melted PEG in the mortar containing the clopidogrel hydrochloride. Stir another 2 minutes, then let the resulting mass cool down to room temperature, before grinding it.

### Example 8 (melt granulate consisting of 50 % by weight of Compritol 888 ATO and 50% clopidogrel hydrochloride)

Melt 10.0 g of Compritol 888 ATO using a 78°C water bath until it has reached 74°C. Place 10.0 g of clopidogrel hydrochloride into a mortar. While manually stirring, pour the melted Compritol into the mortar containing the clopidogrel. Stir another 2 minutes, then let the resulting mass cool down to room temperature, before grinding it.

### Example 9 (storage stability of clopidogrel besylate and melt granulates containing it)

The stability on storage of clopidogrel besylate salt (CLO·Bes) is examined in comparison to melt granulates containing it. The main degradation product is alpha-(2-chlorophenyl)-4,5,6,7-tetrahydrothieno[3,2-c]pyridine-5-acetic acid, hereinafter designated as "ImpA" (ImpA is named according to USP 29 and corresponds to the hydrolysis product of Clopidogrel). In addition, the sum of all impurities ("SumImp") is determined. Both ImpA and SumImp are expressed as percent by weight of the amount of the clopidogrel salt at the beginning of the experiment on day 0 and are determined using the HPLC-system described below.

In the below Table 1 for example the term "10% PEG 6000" means the melt granulate consisting of 10% by weight PEG 6000 and 90% by weight clopidogrel besylate, 30% PEG 6000 means the melt granulate consisting of 30% by weight PEG 6000 and 70% by weight clopidogrel besylate, etc.

**Table 1**

| Tested material | Day 0 | | Day 15 at 50°C and 75% relative humidity | |
|---|---|---|---|---|
| | ImpA [%] | SumImp [%] | ImpA [%] | SumImp [%] |
| CLO·Bes | 0.09 | 0.24 | 0.34 | 0.52 |
| 10% PEG 6000 | 0.08 | 0.23 | 0.21 | 0.39 |
| 30% PEG 6000 | 0.09 | 0.24 | 0.19 | 0.36 |
| 50% PEG 6000 | 0.08 | 0.24 | 0.18 | 0.38 |
| 50% PEG 20000 | 0.09 | 0.25 | 0.19 | 0.38 |
| 50% PEG 35000 | 0.09 | 0.24 | 0.19 | 0.36 |

As evident from the above Table 1 clopidogrel besylate (CLO Bes) contains after storage for 15 days at 50 °C and 75% relative humidity on an average about 79 % (0.34/0.19 = 1.79) more of the impurity ImpA and about 41 % (0.52/0.37 = 1.41) more total impurities SumImp than the clopidogel besylate stabilized in the above melt granulates. The values of 0.19 and 0.37 mentioned herein above in parentheses and used for calculation are the averages of the values listed for the granulates in the columns ImpA and SumImp, respectively, on day 15.

The above-mentioned HPLC system is as follows:
Instruments and operating conditions:
   Column: 250 mm x 4.6 mm Modulo-Cart QS Uptisphere HDO, 3 µm
   Detector: UV Detector or DAD Detector
   Eluent A:0.01M sodium phosphate dibasic aqueous solution (1.78 g Na₂HPO₄ * 2 H₂O/l), adjusted to pH 3.0 ± 0.05 with phosphoric acid 85%
   Eluent B: Acetonitril
   Mobile phase: 35% Eluent A + 65% Eluent B

Gradient:

| minutes | Eluent A [%] | Eluent B %] |
|---|---|---|
| 0.00 | 75 | 25 |
| 0.10 | 65 | 35 |
| 1.50 | 65 | 35 |
| 11.50 | 35 | 65 |
| 40.00 | 20 | 80 |
| 50.00 | 20 | 80 |
| 51.00 | 75 | 25 |

Operating conditions:

| | |
|---|---|
| Flow rate: | 0.4 ml/min |
| Column temperature: | 20°C |
| Injection volume: | 10 µl |
| Wavelength: | 235 nm |
| Runtime: | 65 min |

Retention time:

| Compound | Retention time [minutes] | Relative retention time (vs. clopidogrel) |
|---|---|---|
| Benzenesulfonic acid | 7.6 | 0.18 |
| Diluent | 8.6 | 0.21 |
| Gradient | 11.0 | 0.27 |
| Impurity A | 12.3 | 0.30 |
| Clopidogrel | 41.3 | 1.00 |
| Gradient | 48.0 | 1.16 |

### Preparation of reference and sample solutions:

### Reference solution

Approximately 55.92 mg of clopidogrel besilate reference substance are weighed into a 50 ml volumetric flask. Add 20 ml of mobile phase and sonicate in an ultrasonic bath at ambient temperature until the substance is dissolved (approx. 5 min). Make up to the mark with mobile phase. The concentration of clopidogrel besilate is 0.75 mg/ml as free base. Two independent reference solutions are prepared.

### Sample solution for 75 mg tablets

Weigh 10 tablets and transfer them quantitatively into a 250 ml volumetric flask. Dissolve and make up to the mark with mobile phase. Dilute 25.0 ml to 100.0 ml with mobile phase. Filter through a 0.45 µm PTFE-filter in a HPLC vial. The concentration of clopidogrel is 0.75 mg/ml as free base. Two independent sample solutions are prepared.

### Example 10 (Storage stability of clopidogrel hydrochloride)

The stability on storage of clopidogrel hydrochloride salt (CLO·HCl) has been examined in comparison to melt granulates containing it. The main degradation product is alpha-(2-chlorophenyl)-4,5,6,7-tetrahydrothieno[3,2-c]pyridine-5-acetic acid, hereinafter designated as "ImpA" (impurity acid). In addition, the sum of all impurities ("SumImp") is determined by HPLC analogously as described above for clopidogrel besylate. Both ImpA and SumImp are expressed as percent by weight of the amount of the clopidogrel salt at the beginning of the experiment on day 0. The granulates mentioned in the below Table 2 are melt granulates consisting of 50 % by weight of PEG 6000 and 50% clopidogrel hydrochloride, and 50 % Compritol 888 ATO and 50% clopidogrel hydrochloride, respectively.

**Table 2**

| Tested material | Day 0 | | Day 30 at 40°C and 75% relative humidity | |
|---|---|---|---|---|
| | ImpA [%] | SumImp [%] | ImpA [%] | SumImp [%] |
| CLO·HCl | 0.18 | 0.58 | 1.28 | 1.99 |
| 50% PEG 6000 | 0.13 | 0.49 | 0.65 | 1.03 |
| 50 % Compritol | 0.15 | 0.57 | 0.66 | 1.04 |

As evident from the above Table 2 clopidogrel hydrochloride (CLO HCL) contains after storage for 30 days at 40°C and 75% relative humidity on an average about 95% (1.28/0.655 = 1.95) more of the impurity ImpA (i.e. nearly twice as much) and about 92% (1.99/1.035 = 1.92) more total impurities SumImp than the clopidogrel hydrochloride stabilized in the above melt granulates.

### Example 11 (manufacture of tablets from a melt granulate containing 50% by weight of clopidogrel besylate and 50% by weight of PEG 6000)

In a first step, the granulate is mixed with the excipients in order to form the final blend. 44.74 g of granulate are placed in a container. 26.74 g of Avicel® PH200 (microcrystalline cellulose), 3.80 g of Plasdone® XL (1-ethenylpyrrolidin-2-one) and 0.72 g of Cutina® HR (Hydrogenated Castor Oil) are added. The container is sealed and then the blend is mixed for 10 minutes so that it becomes homogeneous.

In a second step, the final blend is tabletted using 10 mm R9 round, biconvex punches. Each unit has a mass of 380 mg, and is tabletted so that its hardness is between 80 and 100N and its thickness between 5.4 and 6.0 mm.

### Example 12 (manufacture of tablets comprising a melt granulate containing 50% by weight of clopidogrel besylate and 50% by weight of PEG 6000)

In an analogous manner as described in Example 11 tablets are manufactured whereby either the excipients may be added to the melt to form the melt granulate or all or part of the excipients is added to the granulate to be compressed into a tablet. The compositions are given in Table 3. The tablets obtained according to Example 11 or Example 12 are then coated with a composition as given in Table 4.

**Table 3**

| Components | mg / tablet core | mg / tablet core |
|---|---|---|
| Clopidogrel Besylate | 111,85 * | 111,85 * |
| PEG 6000 | 111,85 | 111,85 |
| Avicel PH 200 | 112,50 | 112,50 |
| Plasdone XL | 40,00 | 40,00 |
| Cutina HR | 3,80 | -.- |
| Magnesium stearate | -.- | 3,80 |
| Total | 380,00 | 380,00 |

| | | |
|---|---|---|
| * corresponding to about 75 mg Clopidogrel Base. | | |

**Table 4**

| Coating components per tablet core of 380.0 mg | mg |
|---|---|
| Ethocel STD 7 Premium | 0,50 |
| PEG 6000 | 3,30 |
| Titan dioxide | 2,20 |
| Isopropanol * | 60,00 |
| total coating material on the tablet core | 6,00 |

| | |
|---|---|
| * the isopropanol is evaporated | |

### Example 13

In an analogous manner as described in Example 12 tablets are manufactured whereby either the excipients may be added to the melt to form the melt granulate or all or part of the excipients is added to the granulate to be compressed into a tablet. The compositions are given in Table 5. The tablets obtained are then coated with a composition as given in Table 4. The test results were as follows: ImpA [%] at Day 0: zero (no ImpA);

ImpA [%] at Day 15 (50°C, 75% relative humidity): 0.28.

**Table 5**

| Components | Function | mg / tablet core |
|---|---|---|
| Clopidogrel Besylate | API | 111,85 * |
| PEG 20000 | granulating agent | 37.5 |
| Pharmatose DCL 11 | filler | 206.05 |
| Explocel | disintegrant | 20,00 |
| Magnesium stearate | lubricant | 7.60 |
| Hypromellose | coating agent | 2.00 |
| Titan dioxide | dye | 1.00 |

| | | |
|---|---|---|
| * corresponding to about 75 mg Clopidogrel Base. | | |

## Claims

1. Melt granulate comprising clopidogrel or a pharmaceutically acceptable salt thereof.

2. Melt granulate according to claim 1 comprising a pharmaceutically acceptable salt of clopidogrel.

3. Melt granulate according to claim 1 comprising clopidogrel besylate or clopidogrel hydrochloride.

4. Melt granulate according to any one of claims 1-3 comprising a polyethylene glycol having a mean molecular weight from 1500 to 35000, preferably from 4000 to 10000.

5. Melt granulate according to any one of claims 1-4 containing from 10 to 90 per cent by weight of clopidogrel or a pharmaceutically acceptable salt thereof.

6. Melt granulate according to any one of claims 1-4 containing from 10 to 90 per cent by weight of a pharmaceutically acceptable salt of clopidogrel and (100 minus x) per cent by weight of a polyethylene glycol having a mean molecular weight of from 2000 to 30000, wherein x is the content of the pharmaceutically acceptable salt of clopidogrel expressed as per cent by weight.

7. Melt granulate according to any one of the claims 1-6 in the form of a solid state dispersion of the drug in a pharmaceutically acceptable carrier, wherein said melt granulate comprises a pharmaceutically active compound, preferably clopidogrel, at least one carrier material, and optionally pharmaceutically acceptable excipients.

8. Melt granulate according to claim 7, wherein the optional pharmaceutically acceptable excipients is selected from the group comprising diluents, fillers, binders, disintegrants, coloring agents, flavoring agents, lubricants and preservatives.

9. Melt granulate according to any one of the claims 1 to 8, wherein the ratio of the drug to the pharmaceutically acceptable carrier is within the range of about 10% to 90% of the total solid dispersion weight, more preferable within the range of about 20% to 90%, more preferable within the range of about 40% to 90%, more preferable within the range of about 50% to 90%, of the total dispersion weight.

10. Method of making a melt granulate according to any one of the claims 7-9, **characterized in that** the pharmaceutically acceptable carrier is mixed with the pharmaceutically active compound, and optionally also with the pharmaceutically acceptable excipients, to form an intimate mixture, the obtained mixture is then heated at or near the temperature of the melting point of the pharmaceutically acceptable carrier but below the melting point of the pharmaceutically active compound, thus forming a melt, the obtained melt is then cooled rapidly to provide a congealed mass which optionally is milled to produce a powder.

11. Method of making a melt granulate according to any one of the claims 7-9, **characterized in that** the pharmaceutically acceptable carrier, optionally containing pharmaceutically acceptable excipients, is heated at or near the temperature of the melting point of the pharmaceutically acceptable carrier but below the melting point of the pharmaceutically active compound; the molten pharmaceutically acceptable carrier is then mixed with the pharmaceutically active compound until a homogeneous melt is formed; the obtained melt is then cooled rapidly to provide a congealed mass which optionally is milled to produce a powder.

12. Method according to claim 11, wherein the molten carrier is added to the drug under stirring or mixing.

13. Method according to any one of the claims 10 to 12, wherein the cooling is effected by shock cooling, preferably by adding dry ice (i.e. solid carbon dioxide), preferably in the form of a kind of snow ("dry snow"), or liquefied air, preferably solid carbon dioxide, to the dispersion which thereupon cools rapidly and breaks apart into many small particles and optionally sieving the particles obtained to the desired average grain size.

14. Method according to any one of the claims 10 to 13, wherein the excipients is added to the melt and/or later to the solid state dispersion of the drug.

15. Pharmaceutical composition comprising a melt granulate as claimed in any one of claims 1-9.
